Europäisches Patentamt

European Patent Office

Office européen des brevets

(11) Publication number: **0 138 489**
**B1**

# EUROPEAN PATENT SPECIFICATION

(45) Date of publication of patent specification: **23.12.87**

(51) Int. Cl.⁴: **A 61 M 1/36,** A 61 N 5/06

(21) Application number: **84306667.1**

(22) Date of filing: **28.09.84**

(54) Apparatus and methods for treating cells with radiation.

(30) Priority: **29.09.83 US 537231**
**17.09.84 US 650602**

(43) Date of publication of application:
**24.04.85 Bulletin 85/17**

(45) Publication of the grant of the patent:
**23.12.87 Bulletin 87/52**

(84) Designated Contracting States:
**BE DE FR GB IT**

(56) References cited:
**FR-A-2 426 473**
**GB-A-1 266 242**
**US-A-2 074 909**
**US-A-4 321 919**

(73) Proprietor: **McNeilab, Inc.**
**Springhouse Pennsylvania 19477 (US)**

(72) Inventor: **Taylor, John A.**
**32 Robin Road**
**Furlong PA 18925 (US)**

(74) Representative: **Jones, Alan John et al**
**CARPMAELS & RANSFORD 43 Bloomsbury**
**Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 138 489**

**Description**

This invention relates to the field of treating cells with radiation and specifically relates to the extracorporeal treatment of blood cells with U.V. radiation.

It is well-known that a number of human disease states may be characterized by the overproduction of certain types of leukocytes, including lymphocytes, in comparison to other populations of cells which normally comprise whole blood. Excessive lymphocyte populations result in numerous adverse effects to the patients including the functional impairment of bodily organs eventually resulting in fatality.

US—A—4,321,919 to Edelson describes a method for treating whole blood whereby such populations may be decreased. In general, the method comprises treating the whole blood with dissolved psoralen of a type capable of forming photoadducts with DNA in the presence of U.V.A. radiation in the range of about 320 to 400 nanometers. The covalent bonding that results between the psoralen and the lymphocyte nucleic acid results in lymphocyte metabolic inhibition of the thusly treated cells. These cells are then returned to the patient.

Although this system promises to provide great relief to those patients in life threatening situations, numerous practical problems are yet to be solved. In particular, Edelson fails to provide for a suitable apparatus for applying, in an economical manner, the radiation to the cells.

It is an object of the present invention to provide an apparatus for the practical coupling of radiation to cells which are provided extracorporeally.

It is a related object, that the present invention be simple to produce, and be capable of use by persons having a minimal level of training.

Conventional techniques have relied on a plurality of devices including flasks, columns, spectrophotometer cuvettes, and petri dishes. The sample to be irradiated has been placed in these containers and the container placed adjacent the radiation source. Such systems are limited in that often the radiation wavelengths of particular usefulness fail to penetrate to all of the cells contained within the container due to self-blocking effects and absorption at the outer layers. Further, the systems tend to be batch limited thereby adversely affecting throughput, an important consideration in the clinical environment.

It is an object of the present invention to avoid reliance upon such devices and further, to provide a device and methods for its use which allow continuous treatment and circulation of the cells to be irradiated.

In another class of devices, the sample is passed around a plurality of fibers which optically relay the radiation from the radiation source. In order to be effective, however, these devices require that the fiber optics function very poorly so that the radiation is "lost" through the walls of the fibers, thereby irradiating the cells. Further, such systems are characterized by inefficient energy coupling thereby requiring very large and expensive radiation sources.

It is an object of the present invention to obviate the need for such expensive light sources by providing a comparably far more efficient source to cell energy coupling device.

In accordance with the objects of the present invention, there is provided a device as defined in claim 1 for coupling the radiation provided from commercially available light sources such as the so-called black light fluorescent tubes. In one embodiment, the fluorescent black light source, typically of tubular shape, is coaxially mounted in a tube of larger diameter, the ends of said outer tube being sealed with the radiation source. The outer tube is also provided with an inlet for receiving the cells to be irradiated and an outlet for discharging irradiated cells. In a preferred embodiment, a second outer tube of even larger diameter is coaxially mounted on the previously described device, its ends also in sealing communication with the first outer tube. The second tube further comprises inlet and outlet means for receiving and discharging respectively cells to be irradiated. In the second embodiment, the chamber formed by the inner tube in sealing arrangement with the radiation source serves as a cooling chamber for reducing the heating effects of the bulb.

In the most preferred embodiment, a plurality of the previously described tubes are arranged to form a cassette which is preferably disposable. Flow takes place serially through each of the described tubular arrangements, preferably in a serpentine manner, whereby each tubular apparatus is preferably held vertically and the predominant blood flow therethrough is in a vertical fashion whereby air bubbles and the like are efficaciously flushed out. The embodiment further provides for an inlet and outlet of blood at one end of the cassette and specifically, provides for the blood inlet to be ideally positioned at the bottom most portion of the first tubular apparatus. Thus, the blood outlet advantageously communicates with the top of the last most cylindrical irradiating apparatus.

Thus, body cells to be treated by irradiation with the aforedescribed device are removed from the body and passed through the chamber surrounding the radiation source. The cells may then be returned to the body. The present invention comprises a method for irradiating biological cells without reintroducing the cells into the same body from which they have been removed, as defined in Claim 5. Such treatment may also involve contacting the cells to be irradiated with a compound, such as those available in the psoralen class, which is activated by radiation from the light source. Activated as used herein means that the compound has substantially no effect prior to irradiation, and following irradiation affects the treated cells in a distinguishable and detectable manner. Such a distinguishable and detectable manner may include, for instance, the disruption of membrane integrity and the alteration of DNA within the cell, resulting in the substantial loss of effectiveness or viability of the thusly treated cell.

Further understanding of the present invention may be had by reference to the drawing wherein:

Figure 1 graphically depicts the preferred embodiment;

Figure 2 shows an exploded view of Figure 1;

Figure 3 depicts another embodiment having a single chamber;

Figure 4 depicts the most preferred embodiment of the cassette; and

Figure 5 depicts the instrument in which the Figure 4 apparatus is used.

Although a number of U.V. activated compounds in the psoralen class are known, 8-methoxy psoralen is the compound of choice. An effective radiation for this compound, and many psoralens in general, is the ultraviolet spectrum in the range of approximately 320 to 400 nanometers. A light source which provides such a spectrum (U.V.A) is the Sylvania F8TS/BLB 8W Black Light T5 Blue Bulb. Substantial equivalents are also available from other manufacturers such as General Electric. These bulbs traditionally come in the small "fluorescent" type tube arrangement for which suitable electrical sockets and power supplies are readily available.

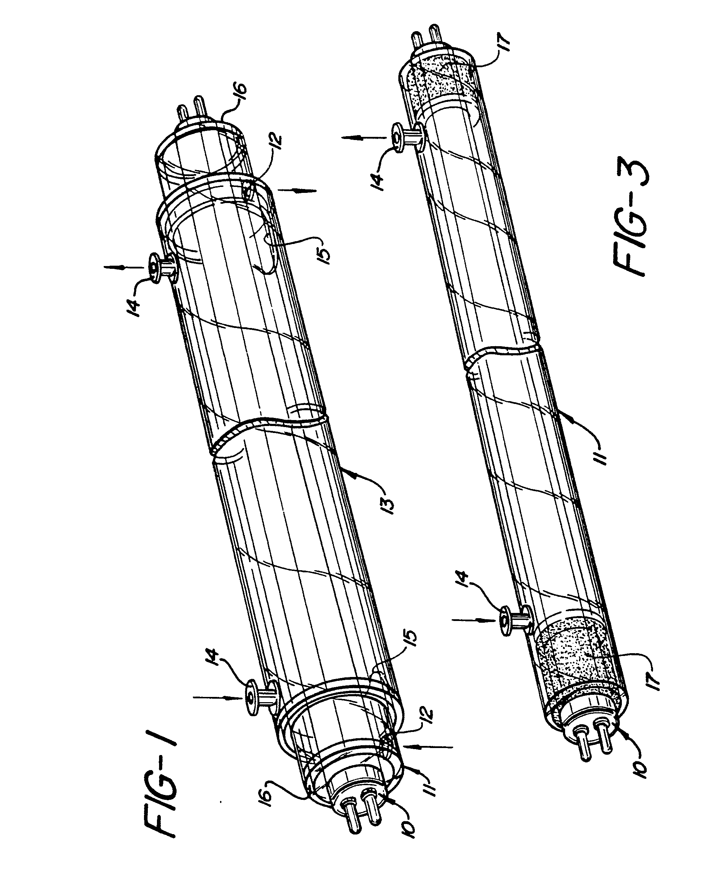

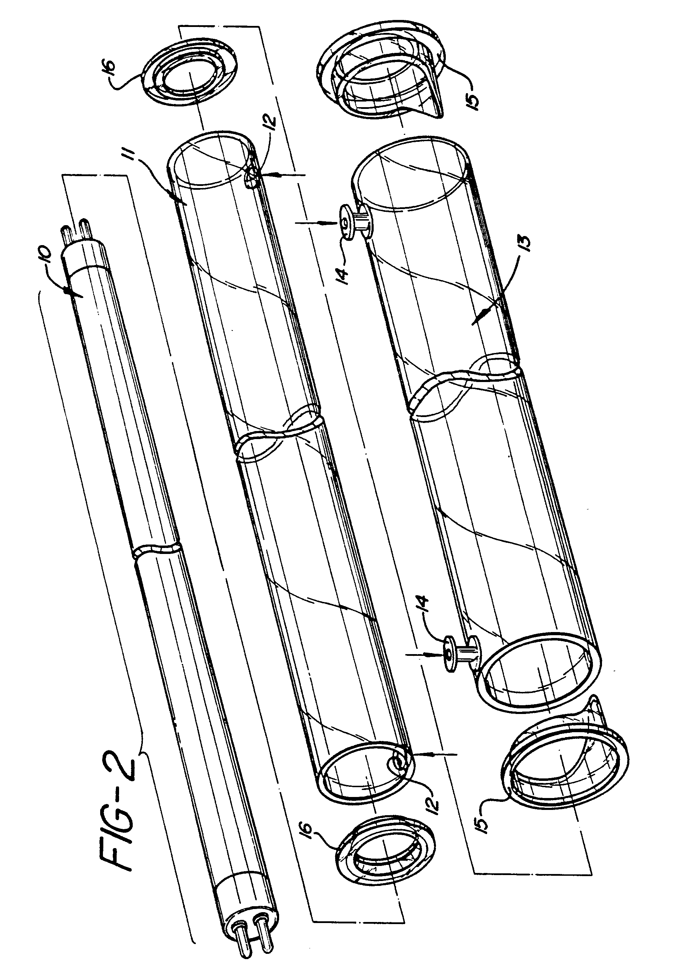

With reference to Fig. 1, such a light source 10 is shown with tubes 11 and 13 coaxially mounted thereon. With additional reference to Fig. 2, it may be readily appreciated that the inner tube 11, is preferably provided with spacer rings 16 mounted on the end thereof in order to maintain coaxial alignment with tube 10. Permanent sealing connection may be made by application of a suitable sealing or cementing material such as the class of epoxy adhesives or solvents such as methylene chloride, particularly useful for "welding" various classes of plastics together. The inner tube is provided with orifices 12 permitting the free flow of convection currents through the chamber thereby cooling the light source 10. Depending on the ambient conditions in which the device is operated, one may find it desirable to increase air flow through the chamber by any suitable means such as forced air and the like.

In the most preferred embodiment, inner tube 11 is made from a substantially U.V. transparent material such as acrylic, cellulose triacetate or a suitable rigid PVC. Based on cost and availability, as well as U.V. transparency, acrylic tends to be the most preferred material and accordingly, is ideally employed in the best embodiment.

With further reference to Figs. 1 and 2, an outer tube 13 is coaxially mounted onto the inner tube 11 advantageously by means of spacers 15. The mounting with the spacers 15 is accomplished in a sealing fashion typically by the application of a suitable solvent for chemically "welding" the outer tube 13, the spacer 15, and the inner tube 11 together. Outer tube 13 has further mounted thereon inlet and outlet 14 for receiving and discharging respectively the fluid sample to be irradiated. In a preferred embodiment, these inlets 14 are Luer type connectors.

It may be further noted that spacers 15 have a protruding "finger" type projection which is preferably disposed on the opposite wall from the inlet 14 and outlet 14. Such a projection advantageously reduces stagnant fluid volumes which would otherwise accumulate in that location. Clearly, such a projection may take the form of virtually any shape and, if desired, may be eliminated all together.

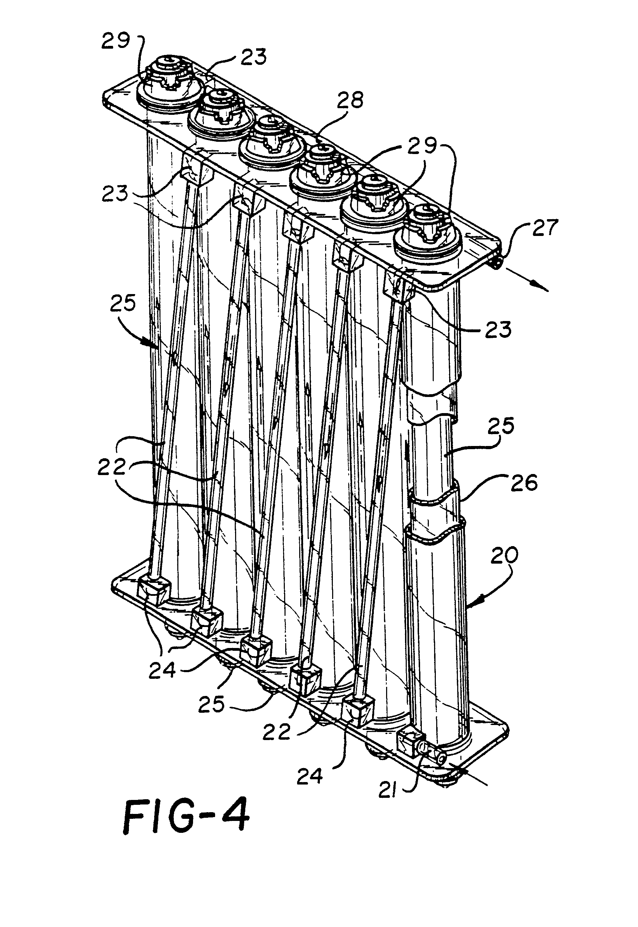

Fig. 3 depicts another embodiment of the instant invention wherein a single chamber, formed by the tube 11 in coaxial orientation with bulb 10 with the ends thereof sealed with a suitable material such as epoxy 17, is used. Suitable in this case would be defined as a material capable of sealingly adhering to the glass of light source 10 and the material 11, and having no substantive interactions with the sample containing cells to be irradiated. Tube 11 is outfitted with inlet and outlets 14 for receiving and discharging the fluid sample. Orientation of flow within the chamber is not important and may be in either direction, however, if a plurality of devices of either the type shown in Fig. 1 or Fig. 3 are employed such as shown in Fig. 4, they are preferably aligned in a vertical fashion and flow is arranged to proceed from bottom to top. Even if a single device is employed, this is the preferred flow arrangement for eliminating air bubbles which might otherwise become trapped in an alternative arrangement.

Fig. 4 shows the most preferred embodiment referred to herein as a cassette. The cassette employs a plurality of irradiating chambers 20 each of which is substantially similar in structure to the embodiment shown in Fig. 1. The ideal arrangement has six such irradiating chambers 20, however, more or less such chambers may be employed, subject only to the constraints of practical construction and ease of handling. It will be readily understood, of course, that increasing the number of irradiation tubes and forcing the blood to flow through each of them, accordingly increases the amount of irradiation to the fluid flowing therethrough.

In the cassette shown in Fig. 4, blood is communicated to the irradiating chamber 20 through inlet 21 which may be either a Luer lock or simply an inlet pipe to which a tubing set may be attached by any suitable means such as solvent welding and the like. The fluid to be irradiated then flows in a generally upward direction and then downwardly through cross connecting means 22 which may comprise a flexible or inflexible tubing connecting chamber outlet 23 to chamber inlet 24 of the next irradiation chamber. This process produces a serpentine flow in the direction indicated by the arrows which has been found to be more effective in evacuating air bubbles and the like. Clearly, the reverse directions may be also employed, as the flow of the fluid through the irradiation chamber 20 is equally effected by the illumination from lamps 25 irregardless of flow. Finally, the fluid reaches outlet 23 of the last irradiation chamber 20 and is emitted from the cassette through the cassette outlet 27. In Fig. 4, this outlet is shown to be at the same side as the cassette inlet 21 via outlet connecting tubing 28. This arrangement facilitates connection and handling as all such connections are made from one end of the cassette. Alternately, the cassette outlet 27 may be in a form similar to cassette inlet 21 directly at the last chamber outlet 23. Each irradiation chamber 20 has tabs 29 holding the irradiation lamp 25 in place within the irradiation chamber. The openings between tabs in combination with inner wall 26 allow the formation of an air space surrounding the lamp 25 which freely communicates with the atmosphere thereby providing cooling in much the same manner as obtained from the interior cooling chamber formed by tube 11 in Fig. 2.

It will be readily understood that although individual cross connecting tubing means 22 are shown in Fig. 4, suitable molding processes may be used whereby such means become an inherent portion of the cassette itself. Such an arrangement would be advantageous from the point of view that fewer connections would have to be made in such instance thereby reducing the opportunity for inadvertent leakages.

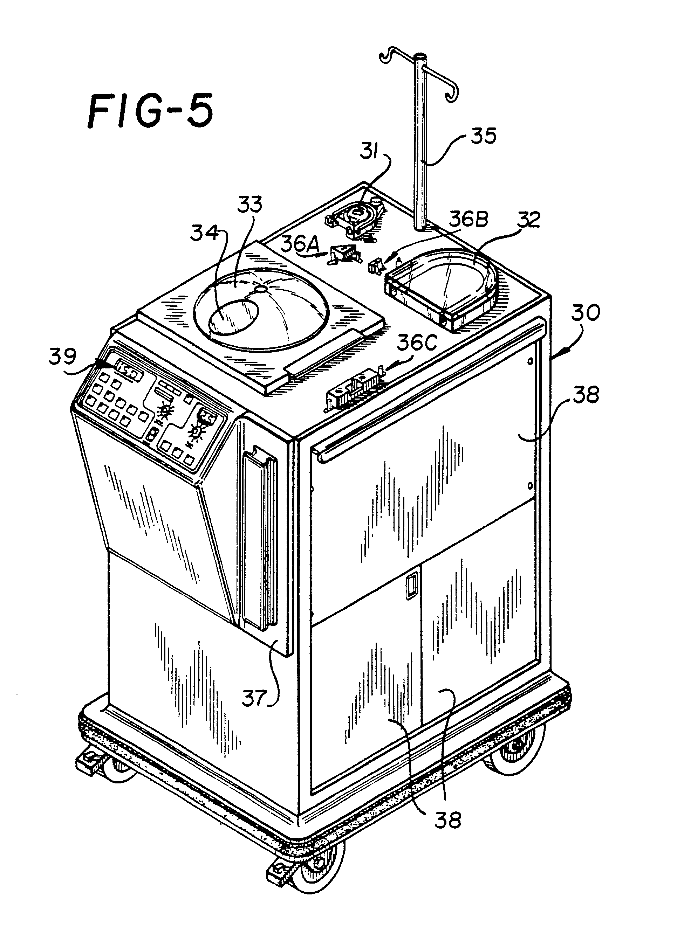

Figure 5 depicts the preferred embodiment of the blood treatment center 30 which employs the cassette of Figure 4 in sliding drawer 37. The flow of heparin or other clot preventing solution through pump 31 via a tubing set (not shown) and the flow of blood through pump 32 from centifuge 33 is controlled by clamps 36A, B and C in turn controlled by control panel 39. The centrifuge 33 is a continuous flow type whereby the operator, by viewing through port 34, determines when a leukocyte rich portion of whole blood is obtained. All solutions are collected in bags (not shown) which hang beside internal access panels 38. After treatment, treated and nontreated blood portions are hung on drip stand 35 and reinfused into the patient.

Example 1

A device as that depicted in Fig. 3, having ends sealed with a combination O ring and epoxy material was employed with a Sylvania F8T5/BLB Lamp. A simple recirculating system was constructed comprising a flexible bag for holding the blood and communicating with inlet located at the bottom of the vertically arranged device. The blood was permitted to flow around the lamp, up through the chamber out the exit orifice whereupon it was returned to the bag. Connections were by means of a flexible tube (Tygon tubing) which was run through a roller bearing type pump. Circuit volume was 150 ml of diluted fresh human blood containing 62 ng/ml of 8-methoxy psoralen. The erythrocyte concentration was $6.7 \times 10^8$ per ml and the leukocyte concentration was $1.4 \times 10^6$ per ml. The priming volume of the device was 25 ml with a blood film thickness of 1.8 mm (0.070 inches) i.e. the distance between the bulb and the inner surface of the surrounding jacket or tube was 1.8 mm (0.07 inches). Blood flow rate was maintained at 50 ml per minute. A pretreatment sample was taken prior to any U.V. exposure and then one sample was run through a circuit such as that described having one device therein and the sample irradiated with 63 joules per ml. An identical system was set up except that 3 tubes in series were employed; flow through each tube being from bottom to top such that 190 joules per ml of irradiation was delivered. The leukocytes were observed for viability over a 4 day period. Viability was determined by treatment with trypsan blue dye and microscopic examination in a manner well-known by those skilled. The results obtained are presented below in Table 1.

TABLE 1
% Leukocyte viability

| Day | Pretreatment | 63 Joules/ml | 190 Joules/ml |
|-----|--------------|--------------|---------------|
| 0 | 100 | 100 | 100 |
| 1 | 100 | 85 | 72 |
| 2 | 97 | 60 | 32 |
| 3 | 95 | 50 | 17 |
| 4 | 93 | 44 | 5 |

(Numbers are given in percentages).

Example 2

Three devices similar to that shown in Fig. 1 were arranged vertically and connected in serial fashion. Connections were made from the output to a Fenwall 350 cc transfer pack with the inlet thereof running through a blood pump and into the first device's inlet. The blood pump maintained flow at 50 ml per minute. The radiation source was a Sylvania F8T5/BLB lamp. Surrounding the lamp was an inner, U.V. transparent tube having a 2.2 cm (7/8") outer diameter. The outer polycarbonate tubing had a 2.54 cm (1") outer diameter. The test circuit was primed for 20 minutes with phosphate buffered saline (PBS) and then drained. The sterilized circuitry containing three devices shown in Fig. 1 was primed with 175 cc of leukophoresced blood from a patient who had orally ingested 8-methoxy psoralen two hours prior to being

4

leukophoresced. The cellular concentrations on this phoresced blood sample were $2.8 \times 10^8$ rbc/ml and $3.8 \times 10^6$ wbc/ml.

The blood was permitted to circulate through the system for 20 minutes at 50 ml per minute without the lamps being turned on. A pretreatment sample $(T_0)$ was taken. The lamps were turned on and the blood circulated for 1 hour and 25 minutes at which time sample $T_1$ was taken. Thereafter, the transfer pack was removed from the circuit the venous and arterial lines were connected together and the blood solution allowed to circulate for another 1 hour 20 minutes at which time sample $T_2$ was obtained.

The effective surface area per cell was calculated to be 163 cm², the effective length 23.5 cm, the priming volume per cell 28 ml, the blood film thickness approximately 1.6 millimeters and the average power per cell 5.5 milliwatts per cm². Sample $T_1$ represented cells irradiated with 94 joules per ml and $T_2$ represented cells irradiated with 190 joules per ml.

Viability of the cells was observed from Day 0 to Day 4. The cells were maintained in vitro by addition of growth medium at a 1:1 proportion of cells to growth medium. Growth medium contained fetal calf serum 30% and 70% RPMI, a well-known tissue culture media.

Results obtained are presented in Table 2 below.

TABLE 2
% Leukocyte viability

| Day | Pretreatment | 94 Joules/ml | 190 Joules/ml |
|-----|--------------|--------------|---------------|
| 0 | 100 | 100 | 100 |
| 1 | 98 | 66 | 54 |
| 2 | 96 | 33 | 7 |
| 3 | 93 | 20 | 4 |
| 4 | 91 | 6 | 2 |

(Numbers are in percentages and represent an average of two readings.)

It will become readily apparent to those skilled that the thickness of the chamber containing the fluid sample, the flow rate of the sample therethrough, lamp power output and cellular concentrations will be advantageously designed in order to afford the desired application of energy to the fluid sample in order to obtain the effects required. Accordingly, the present invention also contemplates the use of any type of radiation source and is not to be limited to the broad band U.V. spectrum employed in the examples.

**Claims**

1. An apparatus for the extracorporeal irradiation of a fluid sample comprising:
a) an elongated radiation source having a first diameter;
b) a first tube having a second diameter larger than said first diameter, and coaxially arranged around said elongated radiation source, said first tube having both ends in sealing engagement with said radiation source and further comprising inlet and outlet means on said first tube communicating with a first chamber formed between the inner surface of the first tube and the outer surface of radiation source.

2. The apparatus as in Claim 1 further comprising a second tube having a third diameter larger than the second diameter, said second tube coaxially arranged with said radiation source and said first tube, means for sealing the ends of said second tube with the outer surface of said first tube thereby forming a second chamber between the outer surface of said first tube and the inner surface of said second tube, said second tube further having inlet and outlet means communicating with said second chamber whereby, air may be caused to pass through either chamber of the device so formed, and fluid may be caused to pass through the other chamber.

3. The apparatus of claim 2, wherein said first chambers communicate with the atmosphere.

4. The apparatus of any one of claims 1 to 3, wherein the radiation source is a visible, infrared, U.V.C., U.V.B., or U.V.A spectrum emitting radiation source.

5. The apparatus of claim 4, wherein the radiation source is a UV spectrum emitting radiation source.

6. The apparatus of any one of claims 1 to 5, wherein the first tube is substantially U.V. transparent.

7. A cassette for the extracorporeal irradiation of a fluid sample comprising a plurality of apparatuses according to any one of claims 1 to 6 and connecting means communicating between the outlet of a first or second tube and the inlet means of the next first or second tube respectively whereby a fluid may be caused to flow through each of said first or second tubes respectively.

8. A method for irradiating biological cells, without reintroducing the cells into the same body from which they have been removed, comprising:

a) providing biological cells to be irradiated extracorporeally;

b) providing an apparatus according to any one of claims 1 to 6;

c) energizing the radiation source; and

d) passing said cells through a chamber of the apparatus.

9. The method of claim 8 further comprising contacting said cells with a compound which is activated by a wavelength of radiation prior to the passing step, and wherein the device has a radiation source selected to provide a wavelength which activates the compound.

10. The method of claim 9, wherein the compound is 8-methoxy psoralen and the radiation source provides a spectrum of U.V. light in the range of about 320—400 nanometers.

11. The method of any one of claims 8 to 10 wherein the biological cells are leukocytes.

12. The method of any one of claims 8 to 11 wherein the passing step comprises passing said biological cells through a cassette according to claim 7, the number of apparatuses in the cassette being chosen in accordance with the radiation source power output, the thickness of the chambers through which the cells pass, and the flow rate of said cells through said chambers whereby an effective energy per ml dose is applied to said cells.

**Patentansprüche**

1. Vorrichtung für die Bestrahlung einer Fluidprobe außerhalb des Körpers, aufweisend:

(a) eine längliche Strahlungsquelle mit einem ersten Durchmesser;

(b) eine erste Röhre, die einen zweiten Durchmesser hat, der größer als der erste Durchmesser ist, und die koaxial um die längliche Strahlungsquelle herum angeordnet ist, wobei die erste Röhre beide Enden in abgedichtetem Eingriff mit der Strahlungsquelle hat und weiterhin Einlaß- und Auslaßeinrichtungen auf der ersten Röhre aufweist, die mit einer zwischen der Innenfläche der ersten Röhre und der Außenfläche der Strahlungsquelle gebildeten ersten Kammer in Verbindung stehen.

2. Vorrichtung nach Anspruch 1, weiterhin aufweisend: eine zweite Röhre mit einem dritten Durchmesser, der größer als der zweite Durchmesser ist, wobei diese zweite Röhre koaxial mit der Strahlungsquelle und der ersten Röhre angeordnet ist, eine Einrichtung für die Abdichtung der Enden der zweiten Röhre mit der Außenfläche der ersten Röhre, wodurch eine zweite Kammer zwischen der Außenfläche der ersten Röhre und der Innenfläche der zweiten Röhre gebildet wird, wobei die zweite Röhre weiterhin Einlaß- und Auslaßeinrichtungen aufweist, die mit der zweiten Kammer in Verbindung stehen, wodurch Luft veranlaßt werden kann, durch eine der beiden Kammern des so gebildet Gerätes zu treten, und Fluid veranlaßt werden kann, durch die andere Kammer zu treten.

3. Vorrichtung nach Anspruch 2, wobei die ersten Kammern mit der Atmosphäure in Verbindung stehen.

4. Vorrichtung nach einem der Ansprüche 1 bis 3, wobei die Strahlungsquelle eine ein sichtbares, Infrarot-, UV C-, UV B- oder UV A-Spektrum emittierende Strahlungsquelle ist.

5. Vorrichtung nach Anspruch 4, wobei die Strahlungsquelle eine ein UV-Spektrum emittierende Strahlungsquelle ist.

6. Vorrichtung nach einem der Ansprüche 1 bis 5, wobei die erste Röhre im wesentlichen UV-durchlässig ist.

7. Kassette für die Bestrahlung einer Fluidprobe außerhalb des Körpers, die eine Vielzahl von Vorrichtung nach einem der Ansprüche 1 bis 6 und eine Verbindungseinrichtung aufweist, die zwischen dem Auslaß einer ersten oder zweiten Röhre und der Einlaßeinrichtung der nächsten ersten bzw. zweiten Röhre eine Verbindung herstellt, wodurch ein Fluid veranlaßt werden kann, durch jede der ersten bzw. zweiten Röhren zu fließen.

8. Verfahren zur Bestrahlung biologischer Zellen, ohne die Zellen wieder in denselben Körper einzuführen, aus dem sie entnommen wurden, aufweisend:

(a) Vorsehen zu bestrahlender biologischer Zellen außerhalb des Körpers;

(b) Vorsehen einer Vorrichtung nach einem der Ansprüche 1 bis 6;

(c) Erregen der Strahlungsquelle; und

(d) Führen der Zellen durch eine Kammer der Vorrichtung.

9. Verfahren nach Anspruch 8, weiterhin aufweisend: In-Kontakt-Bringen der Zellen mit einer Verbindung, die vor dem Durckführschritt durch eine Strahlungswellenlänge aktiviert wird, wobei das Gerät eine Strahlungsquelle hat, die so gewählt wird, daß sie eine Wellenlänge liefert, die die Verbindung aktiviert.

10. Verfahren nach Anspruch 9, wobei die Verbindung 8-Methoxypsoralen ist, und die Strahlungsquelle ein Spektrum von UV-Licht im Bereich von etwa 320—400 Nanometer liefert.

11. Verfahren nach einem der Ansprüche 8 bis 10, wobei die biologischen Zellen Leukozyten sind.

12. Verfahren nach einem der Ansprüche 8 bis 11, wobei der Durchführschritt das Führen der biologischen Zellen durch eine Kassette nach Anspruch 7 aufweist, wobei die Anzahl der Vorrichtung in der Kassette entsprechend der Leistungsabgabe der Strahlungsquelle, der Dicke der Kammern, durch die die Zellen laufen, und der Flußrate der Zellen durch diese Kammern gewählt wird, wodurch eine wirksame Energie/ml-Dosis auf diese Zellen aufgebracht wird.

**0 138 489**

**Revendications**

1. Appareil pour l'irradiation extracorporels d'un échantillon de fluide comprenant:
   a) une source de rayonnement de forme allongée ayant un premier diamètre;
   b) un premier tube ayant un deuxième diamètre plus grand que ledit diamètre et disposé coaxialement autour de ladite source de rayonnement de forme allongée, ledit premier tube ayant deux extrémités qui sont en contact à joint étache avec ladite source de rayonnement, et comprenant en outre des moyens d'entrée et de sortie prévus sur ledit premier tube et qui communiquent avec une première chambre formée entre la surface interne du premier tube et la surface externe de la source de rayonnement.

2. Appareil selon la revendication 1, comprenant en outre un deuxième tube qui possède un troisième diamètre plus grand que le deuxième diamètre, ledit deuxième tube étant disposé coaxialement à ladite source de rayonnement et audit premier tube, des moyens qui scellent les extrémités dudit deuxième tube sur la surface externe du premier tube, en formant de cette façon une deuxième chambre entre la surface externe dudit premier tube et la surface interne dudit deuxième tube, ledit deuxième tube possédant en outre des moyens d'entrée et de sortie qui communiquent avec ladite deuxième chambre, de sorte qu'on peut faire passer de l'air à travers chaque chambre du dispositif ainsi formé et qu'on peut faire passer du fluide à travers l'autre chambre.

3. Appareil selon la revendication 2, dans lequel lesdites premières chambres communiquent avec l'atmosphère.

4. Appareil selon l'une quelconque des revendications 1 à 3, dans lequel la source de rayonnement est une source de rayonnement émettant un spectre visible, infrarouge, U.V.C., U.V.B. ou U.V.A.

5. Appareil selon la revendication 4, dans lequel la source de rayonnement est une source de rayonnement émettant un spectre U.V.

6. Appareil selon l'une quelconque des revendications 1 à 5, dans lequel le premier tube test sensiblement transparent aux U.V.

7. Cassette pour l'irradiation extracorporelle d'un échantillon de fluide, comprenant une pluralité d'appareils selon l'une quelconque des revendications 1 à 6, et des moyens de raccordement qui établissent la communication entre la sortie d'un premier tube ou d'un deuxième tube et les moyens d'entrée du premier tube ou du deuxième tube respectivement suivant, grâce auxquels on peut faire circuler un fluide à travers chacun desdits premier et deuxième tubes respectivement.

8. Procédé pour irradier des cellules biologiques, sand réinjecter les cellules dans le même corps que celui sur lequel elles ont été prises, consistant:
   a) à se munir des cellules biologiques qu'il s'agit d'irradier à l'extérieur du corps:
   b) se munir d'un appareil selon une quelconque des revendications 1 à 6;
   c) alimenter en énergie la source de rayonnement; et
   d) faire passer lesdites cellules à travers une chambre de l'appareil.

9. Procédé selon la revendication 8, consistant en outre à mettre lesdites cellules en contact avec un composé qui est activé par une longueur d'onde de rayonnement avant la phase de circulation et dans lequel le dispositif possède une source de rayonnement choisie de façon à émettre une longueur d'onde qui active le composé.

10. Procédé selon la revendication 9, dans lequel le composé est le 8-méthoxy psoralène et la source de rayonnement émet un spectre de lumière U.V. dans l'intervalle d'environ 320—400 nanomètres.

11. Procédé selon l'une quelconque des revendications 8 à 10, dans lequel les cellules biologiques sont des leucocytes.

12. Procédé selon l'une quelconque des revendications 8 à 11, dans lequel la phase de circulation consiste à faire passer lesdites cellules biologiques à travers une cassette selon la revendication 7, le nombre d'appareils contenus dans la cassette étant choisi en fonction de la sortie de puissance de la source de rayonnement, de l'épaisseur des chambres dans lesquelles passent les cellules et du débit avec lequel lesdites cellules circulent dans lesdites chambres, de manière à appliquer auxdites cellules une dose d'énergie par ml qui soit efficace.

7

0 138 489

FIG-1

FIG-3

FIG-2

0 138 489

FIG-4

FIG-5